# EUROPEAN PATENT SPECIFICATION

(11) **EP 4 051 104 B1**
(45) Date of publication and mention of the grant of the patent: **02.07.2025**
(21) Application number: 20807478.1
(22) Date of filing: 29.10.2020
(51) Int. Cl.: A61B 5/08, A61B 5/00

(54) **METHOD TO DETERMINE COGNITIVE IMPAIRMENT**
METHODE ZUM BESTIMMEN KOGNITIVER BEEINTRÄCHTIGUNGEN
MÉTHODE POUR DÉTERMINER UNE DÉFICIENCE COGNITIVE

(30) Priority: 30.10.2019 GB 201915753
(43) Date of publication of application: 07.09.2022
(73) Proprietor: The University Of Warwick, Coventry CV4 8UW (GB)
(72) Inventor: COVINGTON, James, Coventry CV4 7AL (GB)
(74) Representative: Barker Brettell LLP
(86) International application number: PCT/GB2020/052730
(87) International publication number: WO 2021/084251

(56) References cited:
- US-A1- 2017 254 817
- AKIRA TIELE ET AL: "Breath-based non-invasive diagnosis of Alzheimer's disease: a pilot study", JOURNAL OF BREATH RESEARCH, vol. 14, no. 2, 9 December 2019 (2019-12-09), pages 1 - 26, XP055766581, DOI: 10.1088/1752-7163/ab6016
- MAZZATENTA ANDREA ET AL: "Volatile organic compounds (VOCs) fingerprint of Alzheimer's disease", RESPIRATORY PHYSIOLOGY AND NEUROBIOLOGY, vol. 209, 13 October 2014 (2014-10-13), pages 81 - 84, XP029205405, ISSN: 1569-9048, DOI: 10.1016/J.RESP.2014.10.001
- ULRIKE TISCH ET AL: "Detection of Alzheimer's and Parkinson's disease from exhaled breath using nanomaterial-based sensors", NANOMEDICINE, vol. 8, no. 1, 1 January 2013 (2013-01-01), GB, pages 43 - 56, XP055767439, ISSN: 1743-5889, DOI: 10.2217/nnm.12.105
- H AKMAN ET AL: "Investigation of Correlation between Neurological Diseases and Breath Hexanal", JOURNAL OF BIOANALYSIS & BIOMEDICINE, vol. 10, no. 2, 1 January 2018 (2018-01-01), pages 56 - 59, XP055767515, DOI: 10.4172/1948-593X.1000205

## Description

### Field of the invention

The present invention relates to a method of determining if a subject has a mild cognitive impairment (MCI). The present disclosure also relates to a method of determining if a subject has Alzheimer's disease. The disclosure also extends to a method of determining if a subject is has MCI or AD.

### Background

The increasing lifespan of the human population is inevitably leading to a growing prevalence of neurodegenerative diseases (NDDs). Alzheimer's disease (AD) is a degenerative brain disorder and is the most common cause of dementia. Dementia is characterised by a decline in memory, language, problem-solving, planning, reasoning, and other basic cognitive abilities, which has a direct impact on performing everyday activities. The decline occurs because nerve cells (neurons), in parts of the brain involved in cognitive functions, have been damaged or destroyed. While there is currently no cure or way to stop or slow the progression of dementia, medication, support and care in the disease can help manage symptoms and improve quality of life. In 2017, the worldwide prevalence of dementia was estimated at 50 million people, with a global societal cost of $1 trillion resulting from direct (medical and social care) and indirect (unpaid caregiving by families and friends) costs. The economic impact and costs have been estimated to be greater than that of common chronic diseases, such as heart disease and cancer.

The first and mildest phase of AD is a preclinical phase. This phase is followed by mild cognitive impairment (MCI), which, in turn, is followed by clinically diagnosable AD. The preclinical phase does not include any noticeable clinical signs; however, there are gradual physiological changes at the cellular level, which are associated with the pathogenesis of the disease. MCI is a transitory phase in the progression of AD. Individuals with MCI are defined as those with early memory loss and thinking problems and are identified based on clinical descriptors rather than a diagnosis. Thus, identification of individuals with MCI requires clinical skill and/or experience. For example, identifying individuals with MCI may include taking a history, checking dementia screening bloods, performing a brain scan (CT, MRI or PET) and using neuropsychology testing (e.g. using MoCA, ACE-III and M-ACE sub-score). However, it should be noted that MCI is not unique to AD. Thus, not all individuals who develop a MCI go on to develop AD, some individuals may develop other NDDs, such as Parkinson's disease (PD) or other medical conditions, such as depression or low vitamin levels. Typically, therefore, subjects with MCI who are likely to develop AD are tested for the presence of a biomarker (e.g. amyloid proteins). If, for example, the result of the test for amyloid proteins is positive, then the subject is believed to have a MCI caused by AD. However, from here onwards, the term MCI shall refer to MCI that will may or will go on to become AD.

After developing AD, an individual may go on to develop dementia, which is signified by an impairment of more than one domain of cognitive function and an impairment of the ability to perform activities of daily living, such as getting dressed and household activities. However, from here onwards, the term Alzheimer disease (AD) shall be used to refer to the progression from MCI to an impairment of more than one domain of cognitive function and then to dementia where activities of daily living are becoming impaired. AKIRA TIELE ET AL: "Breath-based non-invasive diagnosis of Alzheimer's disease: a pilot study", JOURNAL OF BREATH RESEARCH, vol. 14, no. 2, 9 December 2019 (2019-12-09), pages 1-26, DOI: 10.1088/1752-7163/ab6016 and US patent application publication US 2017/254817 A1 discloses the breath-based diagnosis of Alzheimer's disease. H AKMAN ET AL: "Investigation of Correlation between Neurological Diseases and Breath Hexanal", JOURNAL OF BIOANALYSIS & BIOMEDICINE, vol. 10, no. 2, 1 January 2018 (2018-01-01), pages 56-59, DOI: 10.4172/1948-593X.1000205 describes the use of breath hexanal in determining neurological diseases.

It is widely accepted that early detection of AD (ideally, in the preclinical phase) will be key to preventing, slowing and stopping the disease. This is when disease-modifying treatments will be most effective. Some research has suggested that the first AD-related brain changes may begin more than 20 years before clinical symptoms emerge. Identifying biomarkers that can reliably identify AD-related developments at an early stage would have a high clinical value, as it could enable early preventative treatments.

There is therefore a need for an improved method of diagnosing and/or distinguishing between different phases in the progression of Alzheimer's disease.

### Statements of the invention

According to a first aspect of the invention, there is provided a method of determining if a subject has a mild cognitive impairment (MCI), the method comprising:
a) measuring, in an exhaled sample that has been taken from a subject, the concentration of one or both VOCs selected from the group consisting of hexanal and heptanal;
b) comparing the concentration measured in a) with a reference concentration; and
c) determining that if the concentration measured in a) is more than about 5% higher than the reference concentration, it may be indicative that the subject has or will develop a MCI.

The inventors have realised that the difference in VOC concentration of an exhaled sample from a subject and a reference concentration (e.g. reference concentration of reference samples from healthy subjects), may be used as a marker that is suggestive that a subject has or is likely to develop a MCI. The invention is therefore advantageous because it will enable clinicians to determine early whether a subject has an MCI. This, in turn, will help the clinician to prevent, slow or stop progression of the disease, or at the very least, improve treatment.

Step (a) may further comprise measuring, in an exhaled sample of a subject, the concentration of one or more further VOCs selected from the group comprising/consisting of 2-propanol, acetone, 2-butanone and 1-butanol.

Determining that the concentration measured in a) is more than about 2.5%, 5%, 10%, 15%, 20%, 25%, 30%, 35%, 40%, 45%, 50%, 55%, 60%, 65%, 70%, 75%, 80%, 85%, 90%, 95% or 100% higher or lower than in the reference concentration, may be indicative that the subject has or will develop a MCI.

In one embodiment, if the concentration of acetone in an exhaled sample is more than about 2.5%, 5%, 10%, 15%, 20%, 25%, 30%, 35%, 40%, 45%, 50%, 55%, 60%, 65%, 70%, 75%, 80%, 85%, 90%, 95% or 100% higher or less than the reference concentration, it may be indicative that the subject has or will develop a MCI. Preferably a higher concentration of acetone than the reference concentration is indicative that the subject has or will develop a MCI.

In one embodiment, if the concentration of 2-butanone in an exhaled sample is more than about 2.5%, 5%, 10%, 15%, 20%, 25%, 30%, 35%, 40%, 45%, 50%, 55%, 60%, 65%, 70%, 75%, 80%, 85%, 90%, 95% or 100% higher or less than the reference concentration, it may be indicative that the subject has or will develop a MCI. Preferably a lower concentration of 2-butanone than the reference concentration is indicative that the subject has or will develop a MCI.

In one embodiment, if the concentration of 2-propanol in an exhaled sample is more than about 2.5%, 5%, 10%, 15%, 20%, 25%, 30%, 35%, 40%, 45%, 50%, 55%, 60%, 65%, 70%, 75%, 80%, 85%, 90%, 95% or 100% higher or less than the reference concentration, it may be indicative that the subject has or will develop a MCI. Preferably a higher concentration of 2-propanol than the reference concentration is indicative that the subject has or will develop a MCI.

In one embodiment, if the concentration of hexanal in an exhaled sample is more than about 2.5%, 5%, 10%, 15%, 20%, 25%, 30%, 35%, 40%, 45%, 50%, 55%, 60%, 65%, 70%, 75%, 80%, 85%, 90%, 95% or 100% higher or less than the reference concentration, it may be indicative that the subject has or will develop a MCI. Preferably a higher concentration of hexanal than the reference concentration is indicative that the subject has or will develop a MCI.

In one embodiment, if the concentration of 1-butanol in an exhaled sample is more than about 2.5%, 5%, 10%, 15%, 20%, 25%, 30%, 35%, 40%, 45%, 50%, 55%, 60%, 65%, 70%, 75%, 80%, 85%, 90%, 95% or 100% higher or less than the reference concentration, it may be indicative that the subject has or will develop a MCI. Preferably a higher concentration of 1-butanol than the reference concentration is indicative that the subject has or will develop a MCI.

In one embodiment, if the concentration of heptanal in an exhaled sample is more than about 2.5%, 5%, 10%, 15%, 20%, 25%, 30%, 35%, 40%, 45%, 50%, 55%, 60%, 65%, 70%, 75%, 80%, 85%, 90%, 95% or 100% higher or less than the reference concentration, it may be indicative that the subject has or will develop a MCI. Preferably a higher concentration of heptanal than the reference concentration is indicative that the subject has or will develop a MCI.

Preferably, step (a) comprises measuring, in an exhaled sample from a subject, the concentration of one or more VOCs selected from the group consisting of hexanal, 2-propanol and heptanal. Most preferably, step (a) comprises measuring, in an exhaled sample from a subject, the concentration of all of the VOCs in the group consisting of hexanal, 2-propanol and heptanal.

Preferably, determining that the concentration of hexanal measured in a) is more than about 100% higher than the reference concentration of hexanal is indicative that the subject has or will develop a MCI. The method according to the first aspect may further comprise determining that if the concentration of hexanal measured in a) is less than about 100% higher than in the reference concentration, it may be indicative that the subject is healthy. The method according to the first aspect may further comprise determining that if the concentration of hexanal measured in a) is between about 100% higher and about 100% lower than the reference concentration, it may be indicative that the subject is healthy.

Preferably, determining that the concentration of 2-propanol in a) is more than about 10% higher than the reference concentration of 2-propanol is indicative that the subject has or will develop a MCI. The method according to the first aspect may further comprise determining that if the concentration of 2-propanol measured in a) is less than about 10% higher than in the reference concentration, it may be indicative that the subject is healthy. The method according to the first aspect may further comprise determining that if the concentration of 2-propanol measured in a) is between about 10% higher and about 10% lower than the reference concentration, it may be indicative that the subject is healthy.

Preferably, determining that the concentration of heptanal in a) is more than about 10% higher than the reference concentration of heptanal is indicative that the subject has or will develop a MCI. The method according to the first aspect may further comprise determining that if the concentration of heptanal measured in a) is less than about 10% higher than, it may be indicative that the subject is healthy. The method according to the first aspect may further comprise determining that if the concentration of heptanal measured in a) is between about 10% higher and about 10% lower than the reference concentration, it may be indicative that the subject is healthy.

According to a second aspect, there is provided a method of determining if a subject has AD, the method comprising:
a) measuring, in an exhaled sample from a subject, the concentration of one or both VOCs selected from the group consisting of acetone and 2-butanone;
b) comparing the concentration measured in a) with a reference concentration; and
c) determining that if the concentration measured in a) is more than about 5% higher or more than about 5% lower than in the reference concentration, it may be indicative that the subject has or will develop AD.

The inventors have realised that the difference in VOC concentration of an exhaled sample from a subject and a reference concentration (e.g. reference concentration of reference samples from healthy subjects), may be used as a marker that is suggestive that a subject has or is likely to develop AD. This is advantageous because it will enable clinicians to determine early whether a subject has AD. This, in turn, will allow clinician to prevent, slow or stop progression of the disease, or at the very least, improve treatment.

Step (a) may further comprise measuring, in an exhaled sample of a subject, the concentration of one or more further VOCs selected from the group comprising/consisting of hexanal, heptanal, 2-propanol and 1-butanol.

Thus, determining that the concentration measured in a) is more than about 2.5%, 5%, 10%, 15%, 20%, 25%, 30%, 35%, 40%, 45%, 50%, 55%, 60%, 65%, 70%, 75%, 80%, 85%, 90%, 95% or 100% higher or lower than in the reference concentration, may be indicative that the subject has or will develop AD.

In one embodiment, if the concentration of acetone in an exhaled sample is more than about 2.5%, 5%, 10%, 15%, 20%, 25%, 30%, 35%, 40%, 45%, 50%, 55%, 60%, 65%, 70%, 75%, 80%, 85%, 90%, 95% or 100% higher or less than the reference concentration, it may be indicative that the subject has or will develop AD. Preferably the concentration of acetone in a subject with AD is higher than the reference concentration.

In one embodiment, if the concentration of 2-butanone in an exhaled sample more than about 2.5%, 5%, 10%, 15%, 20%, 25%, 30%, 35%, 40%, 45%, 50%, 55%, 60%, 65%, 70%, 75%, 80%, 85%, 90%, 95% or 100% higher or less than the reference concentration, it may be indicative that the subject has or will develop AD. Preferably the concentration of 2-butanone in a subject with AD is lower than the reference concentration.

In one embodiment, if the concentration of 2-propanol in an exhaled sample is more than about 2.5%, 5%, 10%, 15%, 20%, 25%, 30%, 35%, 40%, 45%, 50%, 55%, 60%, 65%, 70%, 75%, 80%, 85%, 90%, 95% or 100% higher or less than the reference concentration, it may be indicative that the subject has or will develop AD. Preferably the concentration of 2-propanol in a subject with AD is higher than the reference concentration.

In one embodiment, if the concentration of hexanal in an exhaled sample is more than about 2.5%, 5%, 10%, 15%, 20%, 25%, 30%, 35%, 40%, 45%, 50%, 55%, 60%, 65%, 70%, 75%, 80%, 85%, 90%, 95% or 100% higher or less than the reference concentration, it may be indicative that the subject has or will develop AD. Preferably the concentration of hexanal in a subject with AD is higher than the reference concentration.

In one embodiment, if the concentration of 1-butanol in an exhaled sample is more than about 2.5%, 5%, 10%, 15%, 20%, 25%, 30%, 35%, 40%, 45%, 50%, 55%, 60%, 65%, 70%, 75%, 80%, 85%, 90%, 95% or 100% higher or less than the reference concentration, it may be indicative that the subject has or will develop AD. Preferably the concentration of 1-butanol in a subject with AD is higher than the reference concentration.

In one embodiment, if the concentration of heptanal in an exhaled sample is more than about 2.5%, 5%, 10%, 15%, 20%, 25%, 30%, 35%, 40%, 45%, 50%, 55%, 60%, 65%, 70%, 75%, 80%, 85%, 90%, 95% or 100% higher or less than the reference concentration, it may be indicative that the subject has or will develop AD. Preferably the concentration of heptanal in a subject with AD is higher than the reference concentration.

Preferably, step (a) comprises measuring, in an exhaled sample from a subject, the concentration of one or more VOCs selected from the group consisting of acetone, 2-propanol and 2-butanone. Most preferably, step (a) comprises measuring, in an exhaled sample from a subject, the concentration of all of the VOCs in the group consisting of acetone, 2-propanol and 2-butanone.

Preferably, determining that the concentration of acetone measured in a) is higher than the reference concentration of acetone is indicative that the subject has or will develop AD. Most preferably, determining that the concentration of acetone measured in a) is more than about 5% higher than the reference concentration of acetone is indicative that the subject has or will develop AD. The method according to the second aspect may further comprise determining that if the concentration of acetone measured in a) is less than about 5% higher than in the reference concentration, it may be indicative that the subject is healthy. The method according to the second aspect may further comprise determining that if the concentration of acetone measured in a) is between about 5% higher and about 5% lower than the reference concentration, it is indicative that the subject is healthy.

Preferably, determining that the concentration of 2-propanol measured in a) is higher than the reference concentration of 2-propanol, is indicative that the subject has or will develop AD. Most preferably, determining that the concentration of 2-propanol measured in a) is more than about 10% higher than the reference concentration of 2-propanol, is indicative that the subject has or will develop AD. The method according to the second aspect may further comprise determining that if the concentration of 2-propanol in measured a) is less than about 10% higher than in the reference concentration, it may be indicative that the subject is healthy. The method according to the second aspect may further comprise determining that if the concentration of 2-propanol measured in a) is between about 10% higher and about 10% lower than the reference concentration, it may be indicative that the subject is healthy.

Preferably, determining that the concentration of 2-butanone measured in a) is lower than the reference concentration of 2-butanone is indicative that the subject has or will develop AD. Most preferably, determining that the concentration of 2-butanone measured in a) is more than about 5% lower than the reference concentration of 2-butanone is indicative that the subject has or will develop AD. The method according to the second aspect may further comprise determining that if the concentration of 2-butanone measured in a) is less than about 5% lower than the reference concentration, it may be indicative that the subject is healthy.

According to a third aspect, there is provided a method of determining if a subject is has MCI or AD, the method comprising
a) measuring, in an exhaled sample of a subject, the concentration of the VOC, 1-butanol;
b) comparing the concentration measured in a) with a reference concentration; and
c) determining that if the concentration measured in a) is about 5% to about 60% higher than the reference concentration, it is indicative that the subject has or will develop MCI, or
determining that if the concentration measured in a) is more than about 60% higher than the reference concentration, it may be indicative that the subject has or will develop AD.

The inventors have realised that the difference in VOC concentration of an exhaled sample from a subject and a reference sample (e.g. reference concentration of reference samples from healthy subjects), may be used as a marker that can distinguish between MCI and AD. Thus, the VOC concentration may be used to indicate if a subject has a MCI or AD, or is likely to develop AD. Consequently, this will enable clinicians to provide the most appropriate treatment to prevent, slow or stop the progression of the disease, or at the very least, improve treatment. Step (a) may further comprise measuring, in an exhaled sample of a subject, the concentration of one or more further VOCs selected from the group comprising/consisting of hexanal, heptanal, 2-propanol, acetone and 2-butanone.

In one embodiment, if the concentration of acetone in an exhaled sample subject is about 5% to about 40% higher, about 5% to about 35% higher, about 5% to about 30% higher, about 5% to about 25% higher or about 5% to about 20% higher than the reference concentration, it is indicative that the subject has or will develop AD. If the concentration of acetone in an exhaled sample subject is about 2.5% to about 40% higher, about 5% to about 35% higher, about 10% to about 35% higher, about 15% to about 35% higher or about 20% to about 35% higher than the reference concentration, it is indicative that the subject has or will develop AD. Thus, if the concentration of acetone in an exhaled sample subject is more than about 40%, 35%, 30%, 25% or 20% higher than the reference concentration, it may be indicative that the subject has or will develop MCI.

In one embodiment, if the concentration of 2-butanone in an exhaled sample subject is about 2.5% to about 12% lower or about 5% to about 12% lower than the reference concentration, it is indicative that the subject has or will develop AD. Thus, if the concentration of 2-butanone in an exhaled sample subject is more than about 12% lower than the reference concentration, it may be indicative that the subject has or will develop MCI.

In one embodiment, if the concentration of 2-propanol in an exhaled sample subject is about 5% to about 49% higher, about 5% to about 45% higher, about 5% to about 40% higher, about 5% to about 35%, about 5% to about 30% higher or about 5% to about 25% higher than the reference concentration, it may be indicative that the subject has or will develop MCI. If the concentration of 2-propanol in an exhaled sample subject is about 2.5% to about 49% higher, about 5% to about 45% higher, about 10% to about 45% higher, about 15% to about 45%, about 20% to about 45% higher or about 25% to about 45% higher than the reference concentration, it may be indicative that the subject has or will develop MCI. Thus, if the concentration of 2-propanol in an exhaled sample subject is more than about 49%, 45%, 40%, 35%, 30% or 25% higher than the reference concentration, it may be indicative that the subject has or will develop AD.

In one embodiment, if the concentration of hexanal in an exhaled sample subject is about 5% to about 170% higher, about 5% to about 160% higher, about 5% to about 150% higher, about 5% to about 140% higher, about 5% to about 130% higher, about 5% to about 120% higher, about 5% to about 110% higher, about 5% to about 100% higher, about 5% to about 90% higher, about 5% to about 80% higher, about 5% to about 70% higher, about 5% to about 60% higher or about 5% to about 50% higher than the reference concentration, it may be indicative that the subject has or will develop AD. If the concentration of hexanal in an exhaled sample subject is about 2.5% to about 170% higher, about 5% to about 160% higher, about 10% to about 150% higher, about 15% to about 140% higher, about 20% to about 130% higher, about 25% to about 130% higher, about 25% to about 130% higher, about 30% to about 130% higher, about 35% to about 130% higher, about 40% to about 130% higher, about 45% to about 130% higher, about 50% to about 130% higher or about 55% to about 130% higher than the reference concentration, it may be indicative that the subject has or will develop AD. Thus, if the concentration of hexanal in an exhaled sample subject is more than about 170%, 160%, 150%, 140%, 130%, 120%, 110%, 100%, 90%, 80%, 70%, 60% or 50% higher than the reference concentration, it may be indicative that the subject has or will develop MCI.

In one embodiment, if the concentration of 1-butanol in an exhaled sample subject is about 5% to about 80% higher, about 5% to about 75% higher, about 5% to about 70% higher, about 5% to about 65% higher, about 5% to about 60% higher, about 5% to about 55% higher, about 5% to about 50% higher, about 5% to about 45% higher or about 5% to about 40% higher than the reference concentration, it may be indicative that the subject has or will develop MCI. If the concentration of 1-butanol in an exhaled sample subject is about 2.5% to about 80% higher, about 5% to about 75% higher, about 10% to about 70% higher, about 15% to about 65% higher, about 20% to about 65% higher, about 25% to about 65% higher, about 30% to about 65% higher, about 35% to about 65% higher or about 40% to about 65% higher than the reference concentration, it may be indicative that the subject has or will develop MCI. Thus, if the concentration of 1-butanol in an exhaled sample subject is more than about 80%, 75%, 70%, 65%, 60%, 55%, 50%, 45% or 40% higher than the reference concentration, it may be indicative that the subject has or will develop AD.

In one embodiment, if the concentration of heptanal in an exhaled sample subject is about 5% to about 30% higher, about 5% to about 28% higher, about 5% to about 25% higher, about 5% to about 20% higher or about 5% to about 15% higher than the reference concentration, it may be indicative that the subject has or will develop AD. If the concentration of heptanal in an exhaled sample subject is about 2.5% to about 30% higher, about 5% to about 28% higher, about 10% to about 25% higher or about 15% to about 25% higher than the reference concentration, it may be indicative that the subject has or will develop AD. Thus, if the concentration of heptanal in an exhaled sample subject is more than about 30%, 28%, 25%, 20% or 15% higher than the reference concentration, it may be indicative that the subject has or will develop MCI.

The method according to the third aspect may further comprise determining that if the concentration of 1-butanol measured in a) is less than about 5% higher than in the reference concentration, it may be indicative that the subject is healthy. The method according to the third aspect may further comprise determining that if the concentration of 1-butanol measured in a) is between about 5% higher and about 5% lower than the reference concentration, it may be indicative that the subject is healthy.

Preferably, step (a) comprises measuring, in an exhaled sample of a subject, the concentration of 1-butanol and one or both VOCs selected from the group consisting of hexanal and 2-propanol. Most preferably, step (a) comprises measuring, in an exhaled sample of a subject, the concentration of all of the VOCs in the group consisting of 1-butanol, hexanal and 2-propanol.

Preferably, determining that the concentration of hexanal measured in a) is about 5% to about 100% higher than the reference concentration of hexanal is indicative that the subject has or will develop AD. Preferably, determining that if the concentration of hexanal measured in a) is more than about 100% higher than the reference concentration is indicative that the subject has or will develop MCI. The method according to the third aspect may further comprise determining that if the concentration of hexanal measured in a) is less than about 5% higher than in the reference concentration, it is indicative that the subject is healthy. The method according to the third aspect may further comprise determining that if the concentration of hexanal measured in a) is between about 5% higher and about 5% lower than the reference concentration, it may be indicative that the subject is healthy.

Preferably, determining that the concentration of 2-propanol measured in a) is about 5% to about 49% higher than the reference concentration of 2-propanol is indicative that the subject has or will develop MCI. Preferably, determining that if the concentration of 2-propanol measured in a) is more than about 49% higher than the reference concentration is indicative that the subject has or will develop AD. The method according to the third aspect may further comprise determining that the concentration of 2-propanol measured in a) is less than about 5% higher than in the reference concentration, it is indicative that the subject is healthy. The method according to the third aspect may further comprise determining that if the concentration of 2-propanol measured in a) is between about 5% higher and about 5% lower than the reference concentration, it may be indicative that the subject is healthy.

The skilled person would appreciate that determining that if the concentration of the VOC(s) measured in a) is approximately the same as the reference concentration, it is indicative that the subject is healthy.

Volatile organic compounds may be defined as organic compounds that have a low boiling point at atmospheric pressure. Consequently, they form a liquid phase, which evaporates/diffuses also forms a gaseous phase.

As shown in the Examples, the inventors have surprisingly found that (out of the 3000+ VOCs present in the breath of mammals, such as humans) the concentration of specific VOCs present in exhaled (gaseous) samples is/are indicative of whether a subject is healthy, has a MCI or has AD. Consequently, clinicians can use the invention to ensure that a patient with AD is given the most appropriate treatment (e.g. palliative care and/or medication for the symptoms). The age of onset and rate of progression of AD varies widely from person to person. In light of this, this invention is particularly important in identifying individuals that will rapidly develop or are rapidly developing AD.

Moreover, the invention is non-invasive and, within minutes of a sample being taken, can easily be used to distinguish between healthy individuals, individuals with an MCI and individuals with AD. Thus, the invention is a significant improvement over known methods used to diagnose AD, which, for example, include testing CSF or performing a PET scan. Testing CSF involves invasive step, which require patient compliance, while PET scans are complicated and expensive.

Accordingly, the inventors have realised that the difference in VOC concentration of an exhaled sample from a subject and a reference sample (e.g. reference samples from healthy subjects), may be used as a marker that is suggestive of the presence or absence of an MCI or AD or that a subject is likely to develop or unlikely to develop an MCI or AD.

The skilled person would appreciate that determining that if the concentration of the VOC(s) measured in a) is approximately the same as the reference concentration, it is indicative that the subject is healthy. Thus, for example, the method according to the invention may further comprise determining that if the concentration of one or more of the VOCs in a) is between about 5% higher and about 5% lower than the reference concentration, it is indicative that the subject is healthy.

It will be appreciated that the detection of a single VOC may be used to identify a healthy subject, a subject that has MCI or a subject that has AD. However, the detection of two or more relevant VOCs may provide a more robust diagnosis. When distinguishing between MCI and healthy individuals, hexanal, 2-propanol and heptanal may provide the most robust diagnosis. When distinguishing between AD and healthy individuals, acetone, 2-propanol and 2-butanone may provide the most robust diagnosis. When distinguishing between MCI and AD, hexanal, 2-propanol, and 1-butanol may provide the most robust diagnosis. The method according to the first, second or third aspect of the invention may be complemented by other (known) methods.

It will be appreciated that the absence or presence and/or concentration of a VOC may be determined using any suitable method/technique/technology known in the art, such as gas chromatograph - ion mobility spectrometry (GC - IMS) technology, Gas Chromatograph (GC), Gas Chromatograph - Mass Spectrometry (GCMS), Mass Spectrometry (MS), Ion Mobility Spectrometry (IMS), Differential Mobility Spectrometry (DMS), light absorption Spectrometry, Field Asymmetric Ion Mobility Spectrometry (FAIMS), Electronic Nose, Selective-Ion Flow Tube Mass Spectrometry (SIFT-MS), Protein-transfer-reaction-MS, Optical absorbance/Non-dispersive Infrared and gas sensors (individual or in an array). Preferably, the step of measuring, in an exhaled sample from a subject, the concentration of a VOC comprises detecting the concentration of VOC in a sample by using, for example, GC - IMS technology.

The reference concentration (for distinguishing between a healthy subject and a subject with a MCI or for distinguishing between a healthy subject and a subject with AD) may be based on a reference sample that has been taken from a healthy subject. The reference concentration may have been obtained by assaying a statistically significant number of healthy subjects (e.g. 25 or 50 subjects). Therefore, the reference concentration may be an average concentration, such as the mean, median or mode. Preferably, the average is the mean.

The skilled person would appreciate that in embodiments where the absence or presence and/or concentration of a VOC is determined by GC-IMS, the concentration of a VOC may be equivalent to the intensity of the ion current signal for the VOC (a reference ion intensity).

The reference intensity may be measured in volts. The reference intensity of acetone in a healthy subject may be about 2.66V or within a range of about 2.5V to about 2.9V or about 2.6V to about 2.7V. The reference intensity of 2-butanone in a healthy subject may be about 2.33V or within a range of about 2.2V to about 2.5V or about 2.3V to about 2.5V. The reference intensity of 2-propanol in a healthy subject may be about 1.03V or within a range of about 0.5V to about 1.5V or about 0.75V to about 1.25V. The reference intensity of hexanal in a healthy subject may be about 0.27V or within a range of about 0.1V to about 0.5V or about 0.2V to about 0.4V. The reference intensity of 1-butanol in a healthy subject may be about 0.33V or within a range of about 0.1V to about 0.5V, or about 0.2V to about 0.4V. The reference intensity of heptanal in a healthy subject may be about 0.06V or within a range of about 0.01V to about 0.2V or about 0.03V to about 0.1V.

The method may further comprise determining if the subject has a confounding factor that affects the concentration of the one or more VOCs in an exhaled sample of the subject. The confounding factor may be selected from the group consisting of having a weekly alcohol consumption of 14 units or above, being over 75 years in age, being a smoker and being male. The presence of one or more confounding factors may bias the method according to the invention towards indicating a subject has or will develop a MCI or AD. Thus, the reference concentration(s) are, preferably, taken from healthy subjects that are matched in a selection of confounding factors (having a weekly alcohol consumption of 14 units or above, being over 75 years in age, being a smoker and being male).

The *'subject'* may be a person suspected of having AD or a MCI. The *'subject'* may be a vertebrate, mammal or domestic mammal. Hence, the method according to the invention may be used to diagnose or treat any animal, for example, pigs, cats, dogs, horses, sheep or cows. Preferably, the subject is a human. The subject may be male or female. The term *"healthy"* may refer to an individual without AD or an MCI. Preferably, *"a healthy subject"* is a subject without any known afflictions.

The term *'sample'* may refer to a specimen taken from the body of a subject. The sample may be an exhaled sample from a nose and/or mouth. Preferably, the sample is an exhaled gaseous sample.

The term *'VOC concentration'* may refer to the concentration of one or more VOCs selected from the group consisting of hexanal, 2-propanol, heptanal, acetone, 2-butanone and 1-butanol.

### Figure Legends

For a better understanding of the invention, and to show how embodiments of the same may be carried into effect, reference will now be made, by way of example, to the accompanying Figures, in which:-
**Figure 1** is a 3D topographic map generated by GC-IMS analysis of the constituents of an exhaled breath sample from a healthy individual; and
**Figure 2** is an AUC ROC curve based on the analysis of samples taken from different groups of subjects: Healthy vs MCI (blue); Healthy vs AD (black); MCI vs AD (red). Samples were analysed using a G.A.S. GC-IMS instrument.

### Examples

The inventors demonstrated that exhaled volatile organic compounds (VOCs) in breath can be used as a non-invasive biomarker to distinguish healthy controls from MCI, healthy controls from AD, and MCI from AD. Detection of VOCs was performed using gas chromatography - ion mobility spectrometry (GC-IMS) techniques. Confounding factors, such as age, smoking habits, gender and alcohol consumption were also investigated to demonstrate the efficacy of results.

### Materials and methods

### Subjects

A total of 100 subjects were recruited for this case-control study. Ethical approval was obtained from local research ethics committee (Ref No. 17/18-829, University of Plymouth, UK). MCI and AD patients were recruited by Re:Cognition Health (Plymouth, UK), along with their respective partners as healthy controls. The volunteers and patients received information sheets and were consented following a face-to-face interview by a medical doctor. The study cohort includes 50 patients (25 MCI, 25 AD) and 50 healthy control volunteers. MCI patients were recruited based on the investigator's assessment (inventor S.P.), who reviewed their clinical history and confirmed that the subject demonstrated amnestic symptoms highly suggestive of AD. Thus, MCI subjects were identified by clinical tests of mental function, in line with current practices defined by the NIAAA (National Institute of Aging-Alzheimer's Association, which defines national standards). Similar clinical standards were applied to the AD group as well. Specifically, AD subjects were recruited based on M-ACE scores below 23, a clinical assessment of the presence of amnestic problems, and another domain of cognitive problems, including patients with dementia who had functional impairment. Healthy controls included subjects that had no-known history of neurological disorders (self-reported). The mean age of the MCI and AD group was 74.9 (standard deviation: 7.6), including 29 males and 21 females. An overview of the demographic data of MCI, AD and control subjects is shown in Table 1.

**Table 1 - Demographic data of healthy controls and MCI and AD patients**

| **Parameter** | **MCI (n=25)** | **AD (n=25)** | **Controls (n=50)** |
|---|---|---|---|
| Mean age (SD) | 72.2 (7.3) | 77.5 (7.2) | 71.2 (7.3) |
| Gender ratio M:F | 15:10 | 14:11 | 17:33 |
| Smoking habits | 16 ex-smokers | 10 never smokers | 22 never smokers |
| | 9 never smokers | 12 ex-smokers | 25 ex-smokers |
| | | 3 current smokers | 3 current smokers |
| Alcohol - mean units/week (SD) | 10.8 (11.6) | 3.5 (6.4) | 11.3 (13.4) |
| Medication (No. of Subjects) | Omeprazole (8) | Donepezil (10) | N/A |
| | Atorvastatin (6) | Atorvastatin (6) | |
| | Bisoprolol (5) | Omeprazole (4) | |
| | Paracetamol (4) | Aspirin (4) | |
| | Simvastatin (4) | Amlodipine (4) | |
| | Amlodipine (3) | Folic Acid (4) | |
| | Warfarin (3) | Clopidogrel (3) | |
| | Aspirin (3) | Citalopram (3) | |
| | Citalopram (3) | *47 others* (≤ 2) | |
| | *64 others* (≤ 2) | | |
| Co-diseases / conditions (No. of Subjects) | Hypertension (11) | Hypertension (7) | N/A |
| | Depression (6) | | |
| | High Cholesterol (6) | | |
| | Atrial Fibrillation (4) | High Cholesterol (5) | |
| | Hiatus Hernia (3) | Gastric Reflux (4) | |
| | Migraines (3) | *54 others* (≤ 2) | |
| | Diabetes Mellitus (3) | | |
| | *58 others* (≤ 2) | | |

### Breath Analysis platform

GC-IMS technology was used to detect VOCs in the exhaled breath of subjects. In recent years, there has been a growing presence of portable GC-IMS analysers, which have demonstrated capabilities in medical diagnostics. The BreathSpec (G.A.S., Dortmund, Germany) used, is a commercial instrument, consisting of a gas chromatograph (GC) and an ion mobility spectrometer (IMS). Importantly, the unit analyses at point of care, only takes a few minutes to undertake an analysis and is patient friendly, with breath samples collected without putting strain on the subject. The BreathSpec is equipped with a MXT-200 mid-polarity column (Thames Restek, Saunderton, UK) for gas chromatographic separation, based on chemical interactions with the column.

Thus the chemicals in an exhaled breath sample, including VOCs, are preseparated by the GC column based on their interaction with a retentive layer lining the inside of the column. Thus chemicals, including the VOCs, are eluted from the column at different times (known as the retention time). Following this, the separated chemicals (analytes) are further separated by IMS. The analytes are ionised using a radioactive source and injected into a drift tube, using a shutter grid. The ions drift against a buffer gas under influence of a uniform electric field (400 V/cm), where the various ions achieve different velocities, inversely related to their size, mass and charge. The ions are then collected on a Faraday plate, to produce a time-dependent signal that corresponds with ion mobility. The amount of time taken by an ion to reach the Faraday plate is referred to as the drift time. This technique can measure substances in the low parts-per-billion (ppb) range and delivers measurement results in less than 10 minutes. This unit recirculates and filters ambient air, which allows the unit to operate without the need of an external gas supply.

### Breath sampling

The sampling procedure requires only four seconds of exhaled breath. Subjects were provided with a disposable plastic mouthpiece, which pushes into the mouthpiece holder/sample inlet and connects directly to the front-panel of the instrument. The mouthpieces are open-ended, which allows air inside the mouthpiece to be displaced as exhalation proceeds. As a result, the sampling system can separate out the last portion of exhaled breath - known as end-tidal or alveolar breath. Alveolar breath refers to the last portion (350 mL) of exhaled breath, expelled from within the lungs and the lower-airways, which have undergone gaseous exchange with the blood in the alveoli. Users do not need to exhale until their lungs are as empty as possible and instead are simply asked to breathe normally. This improves reproducibility and makes the device suitable for vulnerable subjects, such as the elderly.

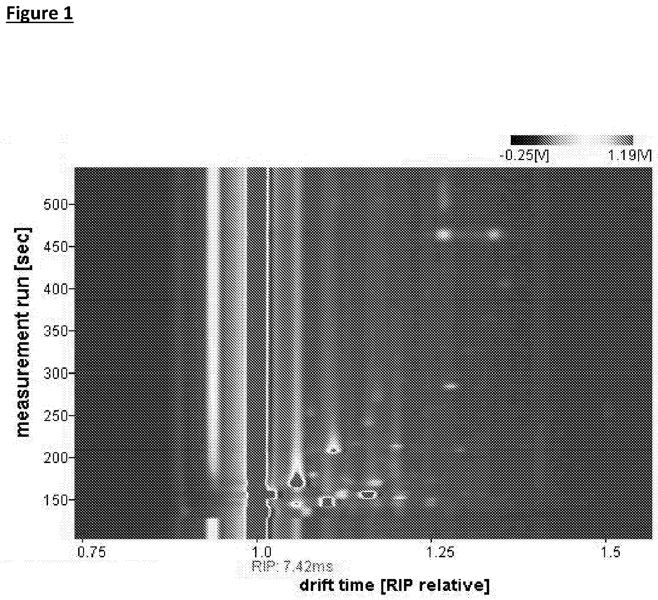

A typical GC-IMS output response to a breath sample (healthy control subject from this study), is shown in Figure 1. The obtained sample is represented in a 3D topographic map, whereby each point is characterised by the retention time in the chromatographic column (in seconds), the drift time (in milliseconds) and the intensity of ion current signal (in millivolts). The signal intensity is indicated by colour, where each high-intensity area represents a single or combination of chemicals (with the same properties). The long line red line is the RIP (reactive ion peak), which is a background signal. Laboratory Analytical Viewer (LAV) software (v2.2.1, G.A.S., Dortmund, Germany) was used for GC-IMS signal viewing.

### Data analysis

The data analysis approach used focuses on distinguishing between the three diagnostic groups: AD, MCI and controls. However, since there are more controls than MCI or AD subjects, the effect of imbalanced datasets needs to be considered. For this reason, a random selection of 25 healthy subjects were used for this analysis. The first step of data analysis involves a pre-processing stage. The aim of this is to reduce the dimensionality, thus leaving data that is non-background. A typical GC-IMS dataset contains 11 million data points, which has high dimensionality, but low information content. Thus, the area of interest is cropped from the centre of the dataset and then a threshold is applied to remove the background. These steps reduce the number of data points by a factor of 100. Following this, a supervised feature selection procedure is undertaken, with class prediction performed using a k-fold cross-validation method (where k=10 in our case). This method involves partitioning the original data set into 10 equally-sized subsets. Of the 10 subsets, a single subset is retained as the validation data for testing the model, and remaining nine subsets are used as training data. Training features are identified using Wilcoxon rank-sum test between the two groups and those feature points with the lowest p-value selected and used to construct models based on five different classifiers. These features are identified purely on a statistical basis and not on any biological function at this stage. This analysis was run using R (version 3.6.0) with standard machine learning sub-packages: support vector machine (SVM) - kernlab; sparse logistic regression (SLR) - glmnet; Gaussian process - gbm, neural network - neuralnet, and random forest (RF) - randomForest. This process is repeated 10 times (number of folds), with each subset used once as validation data. The 10 results are then combined to produce a single estimation and from this, statistical parameters calculated.

In addition to the classification analysis, it is possible to identify unknown VOCs that relate significantly to the efficacy of the generated results. Using GC-IMS Library Search software (v1.0.1, G.A.S., Dortmund, Germany), we can potentially identify compounds based on gas chromatographic retention times and ion mobility drift times, by referencing a NIST database with around 83,000 compound entries. Here, the identified features are plotted back onto the original GC-IMS output and then the chemicals are identified. For quality control, the instrument was normalised to match the GC-IMS Library Search software with the equipped column using a standard ketone mix (2-butanone, 2-pentanone, 2-hexanone, 2-heptanone, 2-octanone and 2-nonanone).

### Confounding factors

Applications of exhaled breath analysis for diagnostic and/or monitoring purposes should consider possible confounding factors. These are factors which are known to have some impact on breath content and can thus introduce bias or generate spurious associations. For example, age is a critical confounder in this study, because increased age is the biggest risk factor for AD. In addition to age, smoking habits, gender and alcohol consumption were considered. The impact of these factors can be evaluated by re-running the classification analysis applied to the diagnostic groups, after re-organising the patients and volunteers based on confounding factors. To simplify the analysis and create more evenly-balanced groups, the confounding groups were defined as: Age [>75 vs <=74 years], smoking habits [never smokers vs ex/current smokers], gender [male vs female] and alcohol consumption [>=14 vs <14 units/week]. The latter threshold represents UK guidelines for regular alcohol consumption. The confounding factor groups are summarised in Table 2.

**Table 2 - Summary of confounding factor groups.**

| **Factor** | **Groups** | **MCI** | **AD** | **Healthy** | **Total** |
|---|---|---|---|---|---|
| Age | <= 74 years | 15 | 8 | 34 | 57 |
| | > 75 years | 10 | 17 | 16 | 43 |
| Smoking | Ex/current smokers | 16 | 15 | 28 | 59 |
| | Never smokers | 9 | 10 | 22 | 41 |
| Gender | Male | 15 | 14 | 17 | 46 |
| | Female | 10 | 11 | 33 | 54 |
| Alcohol | < 14 units per week | 22 | 14 | 31 | 67 |
| | >= 14 units per week | 3 | 11 | 19 | 33 |

### Results

### Example 1 - Chemical identification

VOC analysis indicates that the concentration of three compounds, identified as acetone, 2-propanol and 2-butanone play a crucial role in distinguishing between healthy controls and AD subjects. In the test for AD vs MCI, changes in the concentration of 2-propanol, hexanal and 1-butanol were significant. The separation of healthy controls and MCI relied on changes in the concentration of 2-propanol, hexanal and heptanal.

The observed changes, as measured by GC-IMS, are shown in table 3 with respect to all 100 subjects.

| **VOC** | **AD** | **MCI** | **Controls (intensity of ion current signal in volts)** |
|---|---|---|---|
| Acetone | ↑ 10.5% | ↑↑ 48.4% | 2.66 V |
| 2-Butanone | ↓ -11.4% | ↓ -13.2% | 2.33 V |
| 2-Propanol | ↑↑ 57.6% | ↑ 39.9% | 1.03 V |
| Hexanal | ↑ 52.4% | ↑↑ 209.5% | 0.27 V |
| 1-Butanol | ↑↑ 97.7% | ↑ 11.8% | 0.33 V |
| Heptanal | ↑ 20.9% | ↑ 36.2% | 0.06 V |

### Example 2 - Statistical Analysis

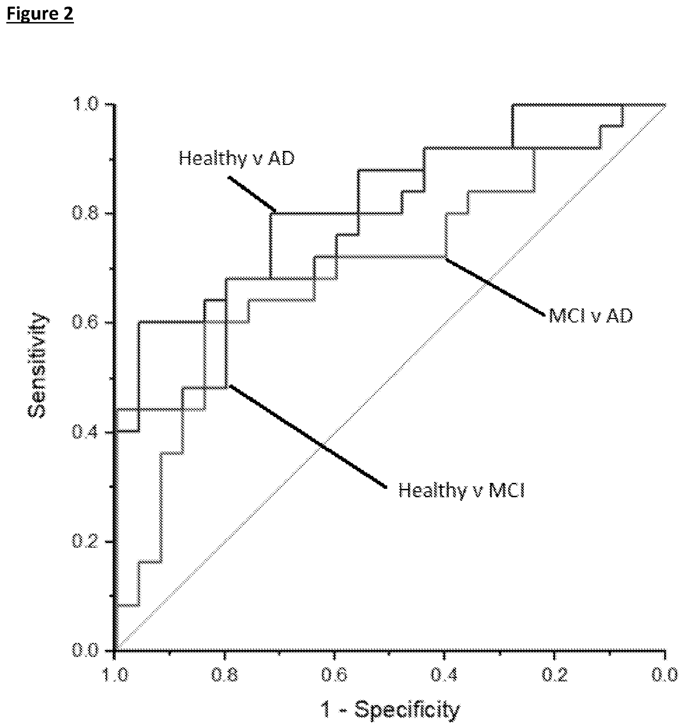

Analysis results are presented in Figure 2 as overlapping receiver operating characteristic (ROC) curves. The corresponding area under curve (AUC) is a measure of how well parameters can distinguish between diagnostic groups, i.e. MCI, AD and healthy. NPV (negative predictive value) and PPV (positive predictive value) for the different comparisons have also been calculated. Analysis results for diagnostic groups were achieved using SLR, and are shown in Table 4.

**Table 4 - GC-IMS diagnostic group results**

| **Test** | **AUC ± 95%** | **Sensitivity** | **Specificity** | **PPV** | **NPV** | ***P*-value** |
|---|---|---|---|---|---|---|
| Healthy vs MCI | 0.77 (0.64 - 0.90) | 0.68 (0.46 - 0.85) | 0.80 (0.59 - 0.93) | 0.77 | 0.71 | 4.10 x 10⁻⁴ |
| Healthy vs AD | 0.83 (0.72 - 0.94) | 0.60 (0.39 - 0.79) | 0.96 (0.80 - 1.00) | 0.94 | 0.71 | 3.48 x 10⁻⁵ |
| MCI vs AD | 0.70 (0.55 - 0.85) | 0.60 (0.39 - 0.79) | 0.84 (0.64 - 0.95) | 0.79 | 0.68 | 0.0076 |

### Example 3 - Confounding factors

The analysis previously conducted on the AD, MCI and healthy groups was repeated, using the same analytical techniques and algorithms, on the confounding factor groups of age, smoking habits, gender and alcohol consumption. The analysis results are summarized in Table 5.

**Table 5 - GC-IMS confounding factors results**

| **Factor** | **AUC ± 95%** | **Sensitivity** | **Specificity** | **PPV** | **NPV** | ***P*-value** |
|---|---|---|---|---|---|---|
| Age [>75 vs <=74 years] | 0.55 (0.43 - 0.67) | 0.70 (0.54 - 0.83) | 0.47 (0.34 - 0.61) | 0.50 | 0.68 | 0.8070 |
| Alcohol [>=14 vs <14 units/week] | 0.60 (0.48 - 0.72) | 0.58 (0.46 - 0.70) | 0.70 (0.51 - 0.84) | 0.80 | 0.45 | 0.0492 |
| Gender [Male vs Female] | 0.54 (0.42 - 0.65) | 0.70 (0.54 - 0.82) | 0.46 (0.33 - 0.60) | 0.52 | 0.64 | 0.2668 |
| Smoking [Ex/current vs Never] | 0.56 (0.44 - 0.68) | 0.81 (0.69 - 0.90) | 0.39 (0.24 - 0.55) | 0.66 | 0.59 | 0.1515 |

Table 5 demonstrates that the possible confounding factors of gender, smoking and age have insignificant influence on breath content. However, alcohol consumption seems to have the most influence on breath, with an AUC of 0.60.

### Discussion

While the exact mechanisms related to AD pathogenesis are not fully understood, there is some evidence to suggest that defects in mitochondrial metabolism (i.e. changes in mitochondrial function and potential dysfunction) play a key role in neurodegeneration. Furthermore, it is believed that mitochondrial dysfunctions in NDDs are associated with increased production of reactive oxygen species (ROS), which cause cell damage and intercellular oxidative stress. Endogenous VOCs (produced in the body) follow metabolic pathways and are transported via the bloodstream to the lungs, where they are exhaled in breath. Oxidative stress has been detected in blood and thus presents an opportunity for the application of breath analysis to facilitate the discovery and evaluation of biomarkers associated with cellular energy metabolism, mitochondrial dysfunction and oxidative stress.

A key advantage of the results within this application is that the ages and genders of the subject cohort are approximately balanced. Furthermore, by recruiting MCI and AD patients with their respective partners as healthy controls, it is possible to design a more robust experiment and minimise the possible effects of lifestyle and environmental factors, which can potentially introduce significant interpersonal differences. Moreover, age-matching is a critical factor to consider, since more women than men have AD and other forms of dementia. For example, almost two-thirds of Americans with Alzheimer's are women. It has been suggested that this discrepancy is due to women generally living longer than men, which increases the risk factor of developing AD. However, there are other factors, such as sex-specific genetic and hormonal factors which can contribute to variance in clinical efficacy. Moreover, lifestyle choices such as smoking, excessive alcohol consumption, poor diet and resulting health conditions (obesity, type-2 diabetes, and cardiovascular disease) can have varied impacts on dementia risk, depending on sex. For this analysis, age was subdivided into 'young elderly' (aged 65-74) and 'older elderly' (aged 75 years or older). This division accounts for the sharp rise in dementia cases over the age of 75 - 15% of those with Alzheimer's are aged 65-75, while 44% are aged 75-85.

VOC analysis indicates that acetone, 2-propanol and 2-butanone contributed significantly to the efficacy of our analysis for AD vs controls. These compounds are generally associated with normal breath. Similarly, changes in other breath markers, such as 2-propanol, hexanal, heptanal and 1-butanol, contributed to separation in AD vs MCI and MCI vs control tests. This suggests that AD-related changes in breath have a subtle impact on overall breath content. Changes in acetone are of particular interest. Early stages of AD are associated with region-specific declines in brain glucose metabolism. This can be supplemented by ketone bodies, including acetoacetate, β-hydroxybutyrate and acetone. These are usually produced from fat stores when glucose is unavailable, e.g. during prolonged fasting or when subscribing to a ketogenic diet, which could lead to increased levels of acetone in the exhaled breath in AD patients. AD patients often suffer loss of appetite, with nearly half of mild-AD subjects reporting appetite changes. The changes observed in exhaled acetone in this study could be related to this phenomenon. To the best of our knowledge, there is currently no known link between 1-butanol and metabolic pathways relating to AD or other NDDs.

Analysis of possible confounding factors indicate that gender, smoking, age and alcohol consumption have insignificant influence of breath content. Of these factors, alcohol seems to have the most influence, with an AUC of around 0.60. However, this factor is not significant enough to create two distinct groups or undermine the AD-related analysis.

### Conclusions

Results from this study confirm the potential utility of analysing breath VOCs to distinguish between MCI, AD and healthy controls. Though this was a simple study, with relatively clean/well defined groups, the approach used was consistently able to separate between diagnostic groups [AUC ± 95%, sensitivity, specificity], Healthy vs MCI: [0.77 (0.64 - 0.9), 0.68, 0.8], Healthy vs AD: [0.83 (0.72 - 0.94), 0.6, 0.96], and MCI vs AD: [0.70 (0.55 - 0.85), 0.6, 0.84]. Analysis of possible confounding factors suggest that gender, age, smoking habits and alcohol consumption had insignificant influence on breath content. VOC analysis indicates that six compounds, tentatively identified as acetone, 2-propanol, 2-butanone, hexanal, heptanal and 1-butanol play a crucial role in distinguishing between diagnostic groups. The GC-IMS analysis technique was shown to be suitable for non-invasive sampling of elderly subjects and demonstrates potential as a fast, high-throughput, real-time diagnostic tool for AD in a point-of-care clinical setting.

## Claims

1. A method of determining if a subject has a mild cognitive impairment (MCI), the method comprising:
a) measuring, in an exhaled sample that has been taken from a body of a subject, the concentration of one or both VOCs selected from the group consisting of hexanal and heptanal;
b) comparing the concentration measured in a) with a reference concentration; and
c) determining that if the concentration measured in a) is more than about 5% higher than the reference concentration, it is indicative that the subject has or will develop a MCI.

2. The method of claim 1, wherein (a) further comprises measuring, in an exhaled sample that has been taken from the body of the subject, the concentration of 2-propanol.

3. The method of claim 1 or claim 2, wherein determining that the concentration of hexanal measured in a) is more than about 100% higher than the reference concentration of hexanal is indicative that the subject has or will develop a MCI.

4. The method of claim 2, wherein determining that the concentration of 2-propanol in a) is more than about 10% higher than the reference concentration of 2-propanol is indicative that the subject has or will develop a MCI.

5. The method according to any one of the preceding claims, wherein determining that the concentration of heptanal in a) is more than about 10% higher than the reference concentration of heptanal is indicative that the subject has or will develop a MCI.

## Patentansprüche

1. Verfahren zum Bestimmen, ob ein Individuum eine leichte kognitive Beeinträchtigung (MCI) aufweist, wobei das Verfahren umfasst:
a) Messen der Konzentration eines oder beider VOCs, die aus der Gruppe ausgewählt sind, die aus Hexanal und Heptanal besteht, in einer Ausatmungsprobe, die von einem Körper eines Individuums genommen wurde;
b) Vergleichen der in a) gemessenen Konzentration mit einer Referenzkonzentration; und
c) Bestimmen, dass, wenn die in a) gemessene Konzentration um mehr als etwa 5 % höher als die Referenzkonzentration ist, dies anzeigt, dass das Individuum eine MCI aufweist oder entwickeln wird.

2. Verfahren nach Anspruch 1, wobei (a) ferner das Messen der Konzentration von 2-Propanol in einer Ausatmungsprobe umfasst, die von dem Körper des Individuums genommen wurde.

3. Verfahren nach Anspruch 1 oder 2, wobei das Bestimmen, dass die in a) gemessene Konzentration von Hexanal um mehr als etwa 100 % höher als die Referenzkonzentration von Hexanal ist, anzeigt, dass das Individuum eine MCI aufweist oder entwickeln wird.

4. Verfahren nach Anspruch 2, wobei das Bestimmen, dass die Konzentration von 2-Propanol in a) um mehr als etwa 10 % höher als die Referenzkonzentration von 2-Propanol ist, anzeigt, dass das Individuum eine MCI aufweist oder entwickeln wird.

5. Verfahren nach einem der vorstehenden Ansprüche, wobei das Bestimmen, dass die Konzentration von Heptanal in a) um mehr als 10 % höher als die Referenzkonzentration von Heptanal ist, anzeigt, dass das Individuum eine MCI aufweist oder entwickeln wird.

## Revendications

1. Méthode permettant de déterminer si un sujet présente un trouble cognitif léger (TCL), le procédé comprenant :
a) la mesure, dans un échantillon expiré prélevé sur le corps d'un sujet, de la concentration d'un ou des deux VOC choisis dans le groupe constitué de l'hexanal et de l'heptanal;
b) la comparaison de la concentration mesurée dans a) avec une concentration de référence ; et
c) la détermination que si la concentration mesurée dans a) est supérieure de plus d'environ 5% à la concentration de référence, cela indique que le sujet a ou développera un TCL.

2. Méthode selon la revendication 1, dans laquelle (a) comprend en outre la mesure, dans un échantillon expiré qui a été prélevé sur le corps du sujet, de la concentration de 2-propanol.

3. Méthode selon la revendication 1 ou la revendication 2, dans laquelle la détermination que la concentration d'hexanal mesurée dans a) est supérieure d'environ 100% à la concentration de référence d'hexanal indique que le sujet a ou développera un TCL.

4. Méthode selon la revendication 2, dans laquelle la détermination que la concentration de 2-propanol dans a) est supérieure d'environ 10% à la concentration de référence de 2-propanol indique que le sujet a ou développera un TCL.

5. Méthode selon l'une quelconque des revendications précédentes, dans laquelle la détermination que la concentration d'heptanal dans a) est supérieure d'environ 10% à la concentration de référence d'heptanal indique que le sujet a ou développera un TCL.
